# EUROPÄISCHE PATENTSCHRIFT

(11) **EP 0 743 299 B1**
(45) Veröffentlichungstag und Bekanntmachung des Hinweises auf die Patenterteilung: **15.09.1999**
(21) Anmeldenummer: 96106952.3
(22) Anmeldetag: 03.05.1996
(51) Int. Cl.: C07C 211/37, C07C 209/62, C07C 265/10, C07C 263/04

(54) **Verfahren zur Herstellung von 2-Fluorcyclopropylaminsulfonsäuresalzen**
Process for the preparation of 2-fluorocyclopropylamine sulfonic acid salts
Procédé pour la préparation de sels d'acides sulfoniques de 2-fluorocyclopropylamine

(30) Priorität: 15.05.1995 DE 19517725
(43) Veröffentlichungstag der Anmeldung: 20.11.1996
(73) Patentinhaber: BAYER AG, 51368 Leverkusen (DE)
(72) Erfinder: Lui, Norbert, Dr., 51061 Köln (DE); Marhold, Albrecht, Dr., 51373 Leverkusen (DE)

(56) Entgegenhaltungen:
- EP-A- 0 191 185
- DE-A- 3 918 792
- J. FLUORINE CHEM., Bd. 49, Nr. 1, 1990, Seiten 127-139, XP000562641 K.R. GASSEN ET AL:
- TETRAHEDRON, Bd. 50, Nr. 13, 1994, Seiten 3889-3904, XP000575878 O. TAMURA ET AL:
- CHEMICAL ABSTRACTS, vol. 116, no. 21, 25.Mai 1992 Columbus, Ohio, US; abstract no. 214046, XP002008675 & JP-A-03 291 258 (DAIICHI SEIYAKU CO LTD) & DATABASE WPI Week 9206 Derwent Publications Ltd., London, GB; AN 92-045984
- "HOUBEN-WEYL, Methoden der Organischen Chemie, Band XI/1, Stickstoffverbindungen II, Seiten 948-954" 1957 , GEORGE THIEME VERLAG , STUTTGART, DE XP002008674

## Beschreibung

Die vorliegende Erfindung betrifft ein vorteilhaftes Verfahren zur Herstellung von 2-Fluorcyclopropylamin-sulfonsäuresalzen ausgehend von 2-Fluorcyclopropyl-carbonsäure.

Aus der JP 03-291 258 A2 ist bekannt, daß man cis-l-(tert.-Butoxycarbonylamin)-2-fluorcyclopropan mit Salzsäure, Schwefelsäure oder Trifluoressigsäure spalten und so die entsprechenden enantiomerenreinen 2 2-Fluorcyclopropylaminsalze erhalten kann. Die Ausbeuten betragen zwischen 51 und 57 % der Theorie. Das benötigte cis-1-(tert.-Butoxycarbonylamin)-2-fluorcyclopropan kann gemäß EP 341 493 A2 aus der entsprechenden Carbonsäure hergestellt werden. Ausbeute-angaben hierzu sind nicht vorhanden. Es muß jedoch eine aufwendige chromatographische Trennung durchgeführt werden.

Aus Tetrahedron 50 (13), 3889-3904 (1994) ist es bekannt N-Benzyl-N-vinyl-carbamate mit Zinkmonofluorcarbenoiden zu N-Benzyl-N-(cis-2-fluorcyclopropyl )-carbamaten umzusetzen und das erhaltene dl-cis-2-Fluorcyclopropylamin mittels L-Mentylchlorameisensäureester einer optischen Resulotion zu unterwerfen. Nachteilig bei diesem Verfahren ist vor allem die schwierige Zugänglichkeit der benötigten Ausgangsprodukte und Reagenzien.

Schließlich ist noch aus J. Fluorine Chem. 49, 127-139 (1990) bekannt Halofluorcarbene, die aus Halofluormethanen mit Basen erhältlich sind, an Butadien oder Isopren zu addieren und so 1-vinylsubstituierte Halofluorcyclopropane zu erhalten. Einige Transformationen wurden damit durchgeführt. Nachteilig bei diesem Verfahren ist die geringe Ausbeute der Carben-Umsetzung.

Es wurde nun ein Verfahren zur Herstellung von 2-Fluorcyclopropylamin-sulfonsäuresalzen ausgehend von 2-Fluorcyclopropylcarbonsäure gefunden, das dadurch gekennzeichnet ist, daß man 2-Fluorcyclopropylcarbonsäure zunächst in das entsprechende Säurechlorid oder Säurebromid überführt, dieses in Gegenwart eines Lösungsmittels mit einem Azid zu einem 2-Fluorcyclopropylisocyanat umsetzt, das 2-Fluorcyclopropylisocyanat nicht aus dem Reaktionsgemisch der Umsetzung mit dem Azid isoliert und abschließend das 2-Fluorcyclopropylisocyanat mit Wasser in Gegenwart einer Sulfonsäure verseift.

Im folgenden wird der Molekülteil 2-Fluorcyclopropyl auch kurz als FCP bezeichnet.

In das erfindungsgemäße Verfahren kann man enantiomerenreine FCP-Carbonsäuren, z.B. (1S, 2S)-, (1R, 2R)-, (1R, 2S)- oder (1S, 2R)-FCP-Carbonsäure oder beliebige Gemische davon einsetzen. Das erfindungsgemäße Verfahren führt zu keinen merklichen Racemisierungen. Man erhält FCP-Amin-Sulfonsäuresalze von praktisch gleicher Chiralität wie die eingesetzte FCP-Carbonsäure.

Die Überführung von FCP-Carbonsäure in das entsprechende Säurechlorid oder -bromid kann auf an sich bekannte Weise, z.B. unter Verwendung von Oxalyl-chlorid, Thionylchlorid oder Phosgen durchgeführt werden (siehe z.B. Houben-Weyl, Band E5, S. 593 bis 615 (1985)).

Die Umsetzung von FCP-Carbonsäurechlorid oder -bromid mit Azid kann z.B. mit Alkaliaziden oder Trialkylsilylaziden durchgeführt werden. Pro Mol FCP-Carbonsäurehalogenid kann man z.B. 0,9 bis 5 Mol eines Azids verwenden. Bevorzugt liegt diese Menge bei 0,95 bis 3 Mol. Die Umsetzung führt man in Gegenwart eines inerten Lösungsmittels durch. Geeignet sind z.B. aromatische, gegebenenfalls halogenierte Kohlenwasserstoffe wie Benzol, Toluol, Xylol, Chlorbenzol, Dichlorbenzol, Chlortoluol und Dichlortoluol und höher siedende Ether wie Dioxan, Tetrahydrofuran, Dimethoxyethan, Glyme und Diglyme. Geeignete Reaktionstemperaturen sind z.B. solche im Bereich 10 bis 130°C. Vorzugsweise arbeitet man bei 50 bis 100°C. Es ist im allgemeinen vorteilhaft, das FCP-Carbonsäurehalogenid und das Lösungsmittel vorzulegen und das Azid in kleinen Portionen zuzufügen. Die Reaktion ist dann beendet, wenn die Gasentwicklung aufhört.

Das gebildete FCP-Isocyanat wird aus dem Reaktionsgemisch nicht isoliert. Man kann für die Verseifung mit Wasser in Gegenwart einer Sulfonsäure direkt das nach der Umsetzung mit dem Azid vorliegende Reaktionsgemisch einsetzen oder ein von überschüssigem Azid z.B. durch Destillation des Azids oder des flüchtigen Teils des Reaktionsgemisches, befreites Reaktionsgemisch.

Als Sulfonsäuren kommen z.B. solche der Formel (I) in Betracht

R-SO₃H (I),

in der
- R: für gegebenenfalls substituiertes C₁-C₁₂-Alkyl, gegebenenfalls substituiertes C₂-C₁₂-Alkenyl, gegebenenfalls substituiertes C₆-C₁₄-Aryl, gegebenenfalls substituiertes C₇-C₁₈-Aralkyl oder gegebenenfalls substituiertes C₇-C₁₈-Alkaryl steht.

Als Substituenten kommen z.B. 1 bis 5 gleiche oder verschiedene Substituenten aus der Gruppe Halogen, Nitro, Cyano, SO₃H und COOR¹ mit R¹ - C₁-C₁₂ -Alkyl oder C₆-C₁₀-Aryl in Frage.

Bevorzugt steht in Formel (I) R für C₁-C₆-Alkyl, C₆-C₁₀-Aryl, C₇-C₁₂-Aralkyl oder C₇-C₁₂-Alkaryl, die gegebenenfalls jeweils mit bis zu 2 gleichen oder verschiedenen Substituenten aus der Gruppe Chlor, Nitro oder SO₃H substituiert sein können. Besonders bevorzugt steht R für p-Tolyl.

Die Sulfonsäuren können auch chiral sein. Beispiele hierfür sind (1R)-(-)-Campher-10-sulfonsäure und (1S)-(+)-Campher-10-sulfonsäure. Eine Enantiomerentrennung kann dann gewünschtenfalls auf der Cyclopropylamin-Stufe vorgenommen werden.

Bezogen auf 1 Mol FCP-Isocyanat kann man z.B. mindestens 1 Mol Wasser und mindestens 0,8 Äquivalente Sulfonsäure einsetzen. Bevorzugt liegen diese Mengen bei 1 bis 50 Mol Wasser und 0,9 bis 3 Äquivalenten Sulfonsäure. Größere Überschüsse an Wasser und/oder Sulfonsäure stören im allgemeinen nicht, sind jedoch nicht wirtschaftlich.

Die Verseifungsreaktion kann z.B. bei Temperaturen im Bereich 0 bis 120°C, vorzugsweise bei 30 bis 70°C, durchgeführt werden.

Als Lösungsmittel für die Verseifung kommen die gleichen in Frage, die oben für die Umsetzung mit Azid beschrieben wurden.

Beispielsweise kann man so vorgehen, daß man die Sulfonsäure und das Wasser vorlegt und das nicht isolierte FCP-Isocyanat und das Lösungsmittel, vorzugsweise das Reaktionsgemisch aus der Umsetzung mit Azid, langsam hinzufügt.

Die Aufarbeitung des nach der Verseifung vorliegenden Gemisches kann z.B. so erfolgen, daß man zunächst die organische Phase abtrennt und daraus das Lösungsmittel zur erneuten Verwendung wiedergewinnt. Aus der wäßrigen Phase kann man dann das Wasser entfernen, z.B durch azeotrope Destillation oder Abdampfen, gegebenenfalls unter vermindertem Druck Wenn keine überschüssige Sulfonsäure im Reaktionsgemisch vorliegt, hinterbleibt dann das hergestellte FCP-Amin-Sulfonsäuresalz häufig schon in hinreichender Reinheit. Eine weitere Reinigung kann z.B. durch Umkristallisation erfolgen. Für eine solche Umkristallisation ist z.B. ein Gemisch aus einem oder mehreren aromatischen Kohlenwasserstoffen und einem oder mehreren aliphatischen Alkoholen geeignet.

Das erfindungsgemäße Verfahren hat eine Reihe von Vorteilen: Es gestattet die Herstellung von FCP-Amin-Sulfonsäuresalzen in hohen Ausbeuten und mit nur geringer Nebenproduktbildung, wobei keine schwierigen Trennungen, beispielsweise durch Chromatographie, nötig sind.

2-Fluorcyclopropylamin, das man aus FCP-Amin-Sulfonsäuresalzen z.B. durch Freisetzung mit einer Base erhalten kann, ist ein Zwischenprodukt zur Herstellung von antibakteriellen Wirkstoffen des Chinolontyps. FCP-Amin-Sulfonsäuresalze sind eine besonders stabile, problemlos lager- und transportfähige Form des 2-Fluorcyclopropylamins.

Die FCP-Amin-Sulfonsäuresalze sind weniger hygroskopisch, als die entsprechenden Hydrochloride. Bei ihrer Herstellung treten keine Schwermetallbelastungen im Produkt und im Abwasser auf, da Sulfonsäuren nicht so korrosiv sind wie die bisher zur Salzbildung verwendeten Säuren, z.B. Salzsäure oder Trifluoressigsäure.

### Beispiele

### Beispiel 1

165 g (1S,2S)-2-Fluorcyclopropancarbonsäure wurden in 300 ml Methylenchlorid bei Raumtemperatur vorgelegt. Dann wurden 220 g Oxalylchlorid langsam zugetropft. Nach Beendigung der Gasentwicklung wurde das Methylenchlorid bei Normaldruck und der Rückstand im Vakuum destilliert. So wurden mit einem Siedepunkt von 38°C bei 13 mbar 183,7 g 2-Fluorcyclopropancarbonsäurechlorid (96 % der Theorie) erhalten.

183 g 2-Fluorcyclopropancarbonsäurechlorid wurden zusammen mit 600 ml Toluol bei 72°C vorgelegt und dann 205 g Trimethylsilylazid zugetropft. Während der Zugabe wurde die Temperatur des Gemisches auf 70 bis 75°C gehalten, anschließend bei bis zu 80°C weitergerührt bis zum Ende der Gasentwicklung. Anschließend wurde das Gemisch auf Raumtemperatur abgekühlt.

305 g p-Toluolsulfonsäuremonohydrat und 500 ml Wasser wurden bei 50°C vorgelegt und das Reaktionsgemisch aus der Umsetzung mit dem Azid (im wesentlichen enthaltend 2-Fluorcyclopropanisocyanat und Toluol) unter Rühren zugetropft. Dann wurde bis zum Ende der Gasentwicklung bei 50°C gerührt. Anschließend wurde die Toluolphase abgetrennt und die wäßrige Phase bis zur Trockene im Vakuum eingeengt. Der feste Rückstand wurde aus Toluol/Ethanol (2:1) umkristallisiert. So wurde in einer Ausbeute von 92 % der Theorie (1R,2S)-2-Fluorcyclopropanamintosylat mit einem Schmelzpunkt von 168 bis 170°C (entsprechend der Literatur) erhalten.

### Beispiel 2

118 g (1R,2R)-2-Fluorcyclopropancarbonsäurechlorid wurden zusammen mit 350 ml Toluol bei 72°C vorgelegt. Dann wurden bei 70 bis 75°C 137 g Trimethylsilylazid zugetropft und abschließend die Mischung bei 80°C bis zum Ende der Gasentwicklung gerührt. Dann wurde auf Raumtemperatur abgekühlt.

196 g p-Toluolsulfonsäuremonohydrat und 350 ml Wasser wurden bei 50°C vorgelegt und dann das Reaktionsgemisch aus der Umsetzung mit dem Azid (im wesentlichen enthaltend 2-Fluorcyclopropylisocyanat und Toluol) unter Rühren zugetropft. Es wurde bis zum Ende der Gasentwicklung bei 50°C gerührt. Dann wurde die Toluolphase abgetrennt und die wäßrige Phase im Vakuum bis zur Trockene eingeengt. Der feste Rückstand wurde aus Toluol/Ethanol (2:1) umkristallisiert. Die Ausbeute an (1S,2R)-2-Fluorcyclopropanamintosylat betrug 88% der Theorie. Der Schmelzpunkt war wie in Beispiel 1 angegeben.

### Beispiel 3

Zu 25 g Natriumazid in 250 ml Toluol wurde bei 90°C eine Lösung von 30 g (1S, 2S)-2-Fluorcyclopropancarbonsäurechlorid in 80 ml Toluol zugetropft. Die Reaktionsmischung wurde bei 85-90°C bis zum Ende der Gasentwicklung gerührt, dann abfiltriert und zu einer Lösung von 47 g p-Toluolsulfonsäure Monohydrat in 100 ml Wasser bei 50°C zugetropft. Es wurde bei dieser Temperatur bis zum Ende der Gasentwicklung gerührt. Die Toluolphase wurde anschließend abgetrennt und die wäßrige Phase bis zur Trockene im Vakuum eingeengt. Der zurückbleibende Feststoff wurde aus Toluol/Ethanol (1:2) umkristallisiert. Die Ausbeute an (1R, 2S)-2-Fluorcyclopropylamintosylat betrug 85 %.

### Beispiel 4

Zu 10 g 2-Fluorcyclopropancarbonsäurechlorid in 50 ml Toluol wurden bei 80°C 11 g Trimethylsilylazid zugetropft. Die Reaktionsmischung wurde bei 85-90°C bis zum Ende der Gasentwicklung gerührt und zu einer Lösung von 20,8 g (1R)-(-)-Campher-10-sulfonsäure in 80 ml Wasser bei 50°C zugetropft. Es wurde bei dieser Temperatur bis zum Ende der Gasentwicklung gerührt. Die Toluolphase wurde anschließend abgetrennt und die wäßrige Phase bis zur Trockene im Vakuum eingeengt. Der zurückbleibende Feststoff wurde aus Toluol/Ethanol umkristallisiert. Die Ausbeute an 2-Fluorcyclopropylamintosylat betrug 88 %.

¹H-NMR (DMSO): δ 0,8 (s, 3H); 1.08 (s, 3H); 1,1-1,5 (m, 4H); 1,8-2,1 (m, 3H); 2,3 (m, 1H); (m, 3H); 3,1 (d, 1H); 4,9 (m, 1H, J _{HF} = 64 Hz); 8,6 (s, 3H).

## Patentansprüche

1. Verfahren zur Herstellung von 2-Fluorcyclopropylamin-sulfonsäuresalzen ausgehend von 2-Fluorcyclopropylcarbonsäure, dadurch gekennzeichnet, daß man 2-Fluorcyclopropylcarbonsäure zunächst in das entsprechende Säurechlorid oder Säurebromid überführt, dieses in Gegenwart eines Lösungsmittels mit einem Azid zu einem 2-Fluorcyclopropylisocyanat umsetzt, das 2-Fluorcyclopropylisocyanat nicht aus dem Reaktionsgemisch der Umsetzung mit dem Azid isoliert und abschließend das 2-Fluor-cyclopropylisocyanat mit Wasser in Gegenwart einer Sulfonsäure verseift.

2. Verfahren nach Anspruch 1, dadurch gekennzeichnet, daß man (1S,2S)-, (1R,2R)-, (1R,2S)- oder (1S,2R)-2-Fluorcyclopropancarbonsäure oder beliebige Gemische davon einsetzt.

3. Verfahren nach Ansprüchen 1 bis 2, dadurch gekennzeichnet, daß man als Azid Alkaliazide oder Trialkylsilylazide in einer Menge von 0,9 bis 5 Mol pro Mol 2-Fluorcyclopropylcarbonsäurehalogenid einsetzt.

4. Verfahren nach Ansprüchen 1 bis 3, dadurch gekennzeichnet, daß man eine Sulfonsäure der Formel (I) verwendet
R-SO₃H (I),
in der
R für gegebenenfalls substituiertes C₁-C₁₂-Alkyl, gegebenenfalls substituiertes C₂-C ₁₂-Alkenyl, gegebenenfalls substituiertes C₆-C₁₄-Aryl, gegebenenfalls substituiertes C₇-C₁₈-Aralkyl oder gegebenenfalls substituiertes C₇-C₁₈-Alkaryl steht.

5. Verfahren nach Ansprüchen 1 bis 4, dadurch gekennzeichnet, daß die Sulfonsäure 1 bis 5 gleiche oder verschiedene Substituenten aus der Gruppe Halogen, Nitro, Cyano, SO₃H und COOR¹ mit R¹ = C₁-C₁₂-Alkyl, oder C_{1,}-C₁₀-Aryl enthält.

6. Verfahren nach Ansprüchen 1 bis 5, dadurch gekennzeichnet, daß man pro Mol 2-Fluorcyclopropylisocyanat mindestens 1 Mol Wasser und mindestens 0,8 Äquivalente Sulfonsäure einsetzt und bei 0 bis 120°C arbeitet.

7. Verfahren nach Ansprüchen 1 bis 6, dadurch gekennzeichnet, daß man aus dem nach der Verseifung vorliegenden Reaktionsgemisch zunächst die organische Phase abtrennt und dann aus der wäßrigen Phase das Wasser entfernt.

8. Verfahren nach Ansprüchen 1 bis 7, dadurch gekennzeichnet, daß man eine chirale Sulfonsäure einsetzt.

## Claims

1. Process for the preparation of 2-fluorocyclopropylamine sulphonic acid salts, starting from 2-fluorocyclopropylcarboxylic acid, characterized in that 2-fluorocyclopropylcarboxylic acid is first converted into the corresponding acid chloride or acid bromide, this is reacted with an azide in the presence of a solvent to give a 2-fluorocyclopropyl isocyanate, the 2-fluorocyclopropyl isocyanate is not isolated from the reaction mixture of the reaction with the azide and finally the 2-fluorocyclopropyl isocyanate is saponified with water in the presence of a sulphonic acid.

2. Process according to Claim 1, characterized in that (1S,2S)-, (1R,2R)-, (1R,2S)- or (1S,2R)-2-fluorocyclopropanecarboxylic acid or any mixtures thereof are used.

3. Process according to Claims 1 to 2, characterized in that alkali metal azides or trialkylsilyl azides are used as azide in an amount of 0.9 to 5 mol per mole of 2-fluorocyclopropylcarbonyl halide.

4. Process according to Claims 1 to 3, characterized in that a sulphonic acid of the formula (I) is used
R-SO₃H , (I),
in which
R represents unsubstituted or substituted C₁-C₁₂-alkyl, unsubstituted or substituted C₂-C₁₂-alkenyl, unsubstituted or substituted C₆-C₁₄-aryl, unsubstituted or substituted C₇-C₁₈-aralkyl or unsubstituted or substituted C₇-C₁₈-alkaryl,

5. Process according to Claims 1 to 4, characterized in that the sulphonic acid contains 1 to 5 identical or different substituents from the group consisting of halogen, nitro, cyano, SO₃H and COOR¹, where R = C₁-C₁₂-alkyl or C₆-C₁₀-aryl.

6. Process according to Claims 1 to 5, characterized in that, per mole of 2-fluorocyclopropyl isocyanate, at least 1 mol of water and at least 0.8 equivalents of sulphonic acid are used and the procedure is carried out at 0 to 120°C.

7. Process according to Claims 1 to 6, characterized in that the organic phase is first separated off from the reaction mixture present after the saponification and then the water is removed from the aqueous phase.

8. Process according to Claims 1 to 7, characterized in that a chiral sulphonic acid is used.

## Revendications

1. Procédé pour la préparation de sels d'acide sulfonique de 2-fluorocyclopropylamine à partir d'acide 2-fluorocyclopropylcarboxylique, caractérisé en ce que l'on transforme tout d'abord l'acide 2-fluorocyclopropylcarboxylique en le chlorure d'acide ou en le bromure d'acide correspondant, on transforme celui-ci en présence d'un solvant avec un azide en un isocyanate de 2-fluorocyclopropyle, on n'isole pas l'isocyanate de 2-fluorocyclopropyle du mélange réactionnel de la réaction avec l'azide et on saponifie finalement l'isocyanate de 2-fluorocyclo-propyle avec de l'eau en présence d'un acide sulfonique.

2. Procédé selon la revendication 1, caractérisé en ce que l'on utilise l'acide (1S,2S)-, (1R,2R)-, (1R,2S)- ou (1S,2R)-2-fluorocyclopropanecarboxylique ou des mélanges quelconques de ceux-ci.

3. Procédé selon les revendications 1 à 2, caractérisé en ce que l'on utilise comme azide des azides d'alcalis ou des azides de trialkylsilyles dans une quantité de 0,9 à 5 mol par mol d'halogénure d'acide 2-fluorocyclopropylcarboxylique.

4. Procédé selon les revendications 1 à 3, caractérisé en ce que l'on utilise un acide sulfonique de la formule (I)
R-SO₃H , (I),
dans laquelle
R représente un groupe alkyle en C₁-C₁₂ éventuellement substitué, un groupe alcényle en C₂-C₁₂ éventuellement substitué, un groupe aryle en C₆-C₁₄ éventuellement substitué, un groupe aralkyle en C₇-C₁₈ éventuellement substitué ou un groupe alkaryle en C₇-C₁₈ éventuellement substitué.

5. Procédé selon les revendications 1 à 4, caractérisé en ce que l'acide sulfonique contient de 1 à 5 substituants identiques ou différents choisis parmi un halogène, un groupe nitro, cyano, SO₃H et COOR¹ avec R¹ = groupe alkyle en C₁ -C₁₂ ou groupe aryle en C₆-C₁₀.

6. Procédé selon les revendications 1 à 5, caractérisé en ce que l'on utilise par mol d'isocyanate de 2-fluorocyclopropyle au moins une mol d'eau et au moins 0,8 équivalent d'acide sulfonique et on travaille à de 0 à 120°C.

7. Procédé selon les revendications 1 à 6, caractérisé en ce que l'on sépare d'abord la phase organique du mélange réactionnel présent après la saponification et on élimine ensuite l'eau de la phase aqueuse.

8. Procédé selon les revendications 1 à 7, caractérisé en ce que l'on utilise un acide sulfonique chiral.
